# EUROPEAN PATENT APPLICATION

(11) **EP 4 748 934 A1**
(43) Date of publication of application: **27.05.2026**
(21) Application number: 24214490.5
(22) Date of filing: 21.11.2024
(51) Int. Cl.: C12N 15/115, A61K 31/7088

(54) **DNA APTAMER FOR INHIBITING FGFR1**

(71) Applicant: Masarykova Univerzita, 60177 Brno (CZ)
(72) Inventor: Krejci, Pavel, Brno (CZ); Feketova, Zuzana, Brno (CZ); Trantirek, Lukas, Brno (CZ); Zlinska, Vladimira, Brno (CZ)
(74) Representative: Harber IP s.r.o.

(57) **Abstract**

The present invention provides a DNA aptamer having a length of up to 80 nucleotides, preferably up to 40 nucleotides, and being or containing the sequence:
GGGATACAGGGCTXTGTCTATGGTGTGGATGGCGGATACC (SEQ ID NO. 1), wherein X is T or A, and wherein one to three deoxynucleotides may optionally be modified by fluorination. The DNA aptamers according to the invention are selective FGFR1 inhibitors and are useful in the treatment of skeletal disorders, developmental disorders, and cancers caused by aberrant FGFR1 signaling.

## Description

### Field of Art

The present invention relates to a DNA aptamer for specifically inhibiting fibroblast growth factor receptor 1 (FGFR1), suitable for treatment of growth disorders and cancer.

### Background Art

Impaired fibroblast growth factor receptor (FGFR) signaling is associated with several human conditions, including skeletal disorders and cancer. Current therapeutic targeting of FGFR signaling relies on small molecule inhibitors of FGFR tyrosine kinase activity (TKIs). However, FGFR TKIs are limited in their target specificity as they generally inhibit all four FGFRs (FGFR1-4) as well as other FGFR-unrelated receptor tyrosine kinases.

The fibroblast growth factor (FGF) family consists of 18 proteins that act as morphogens, growth factors and metabolic hormones to regulate important physiological processes during development and life. FGFs signal via transmembrane FGF-receptor tyrosine kinases (FGFRs). In mammals, there are four FGFRs (FGFR1-4) that further diversify into the "b" or "c" variants by alternative splicing in the extracellular Ig-like domain 3, increasing the number of functionally active FGFR variants to seven (FGFR1b, FGFR1c, FGFR2b, FGFR2c, FGFR3b, FGFR3c, FGFR4). Since the Ig3 domain is involved in ligand binding, the seven FGFR variants show differences in the selection of their cognate FGFs. In mammals, FGF-FGFR interactions participate in the majority of developmental processes, including blastocyst formation, gastrulation, and morphogenesis of lungs, limbs, and brain (Yamaguchi and Rossant, 1995, https://doi.org/10.1016/0959-437X(95)90053-J; Naski and Omitz, 1998, doi: 10.2741/a321. PMID: 9683641). Impaired FGFR signaling is associated with many pathological conditions, including growth disorders and cancer (Gallo et al., 2015, https://doi.org/10.1016/j.cytogfr.2015.03.003).

Current therapeutic approaches for targeting FGFR signaling are based on small molecule inhibitors of FGFR catalytic activity (tyrosine kinase inhibitors, TKIs). Fifteen TKIs are currently in clinical trials for tumors caused by lesions in FGFR genes, such as FGFR amplifications, activating mutations and fusion oncogenes involving FGFRs. These TKIs inhibit the tyrosine kinase activity of FGFRs by preventing ATP binding to the FGFR kinase domain, with the exception of futibatinib and RLY-4008, which form a covalent bond with the cysteine residue in the phosphate binding loop of the FGFR kinase domain. Most of the available FGFR TKIs exhibit low specificity among the FGFR1-4 variants and therefore inhibit most of the 62 FGF-FGFR interactions *in vivo.* This is due to the conservation of the FGFR tyrosine kinase domains targeted by the TKIs. In addition, FGFR TKIs also inhibit non-FGFR receptor tyrosine kinases (RTKs), including those belonging to the VEGFR, PDGFR, DDR and MET RTK families. The low specificity among FGFRs and off-target activity may be responsible for the side effects and toxicity of FGFR-TKI therapy, thus limiting its use.

Recently, aptamer technology has yielded more specific inhibitors of FGFR signaling. For example, iR3, an RNA aptamer that specifically inhibits FGFR3 (Kamatkar et al., 2019, https://doi.org/10.1016/j.omtn.2019.06.020), Apt_46, a DNA aptamer that inhibits FGFR2b but not the other FGFR variants (Eguchi et al. 2021, https://doi.org/10.1021/jacsau.0c00121), or RBM-007, an RNA aptamer that neutralizes the FGF ligand FGF2 (Kimura et al, 2021, https://doi.org/10.1126/scitranslmed.aba4226; Nakamura, 2021, https://doi.org/10.3390/cells10071617). To inventors' best knowledge, there is currently no specific aptamer-based inhibitor specifically inhibiting FGFR1.

### Summary of the Invention

The present invention provides a DNA aptamer having a length of up to 80 nucleotides, preferably up to 40 nucleotides, and being or containing the sequence:
GGGATACAGGGCTXTGTCTATGGTGTGGATGGCGGATACC (SEQ ID NO. 1),
wherein X is T or A, and wherein one to three deoxynucleotides may optionally be modified by fluorination.

Preferably, the modification by fluorination is a modification wherein the deoxyguanosine in position 9 and/or 32 of SEQ ID NO. 1 are optionally replaced by 2'-deoxy-2'-fluoroarabinoside guanosine.

Preferably, the DNA aptamer according to the invention is or contains a sequence selected from the group:
GGGATACAGGGCTTTGTCTATGGTGTGGATGGCGGATACC (SEQ ID NO. 2), referred to as VZ23 in the following text, and
GGGATACAGGGCTATGTCTATGGTGTGGATGGCGGATACC (SEQ ID NO. 3), referred to as VZ23-T14A in the following text, and
GGGATACAG_{F}GGCTTTGTCTATGGTGTGGATGGCGGATACC (SEQ ID NO. 4), referred to as G9F in the following text, and
GGGATACAGGGCTATGTCTATGGTGTGGATGG_{F}CGGATACC (SEQ ID NO. 5), referred to as G32F in the following text, and
ATACCAGCTTATTCAATTGGGATACAGGGCTTTGTCTATGGTGTGGATGGCGGATACCAG ATAGTAAGTGCAATCT (SEQ ID NO. 6), referred to as "long VZ23" in the following text. The G_{F} in the sequences G9F and G32F corresponds to 2'-deoxy-2'-fluoroarabinoside guanosine.

The DNA aptamers of the invention are single-stranded DNA molecules that adopt a specific three-dimensional structure in the presence of a physiological concentration of Mg²⁺ ([Mg²⁺] ≥ 1 mM), binding selectively to FGFR1 (both FGFR1b and FGFR1c) but not binding to FGFR2b, FGFR2c, FGFR3b or FGFR4, and mediating specific and efficient inhibition of FGFR1.

The present invention further provides a composition comprising at least one DNA aptamer of the invention, at least one pharmaceutically acceptable excipient, and optionally Mg²⁺ ions. The pharmaceutically acceptable excipients and their use are well known to a person skilled in the art of pharmaceutical formulations. In some embodiments, the pharmaceutically acceptable excipients may include solvents, diluents, fillers, binders, glidants, lubricants, preservatives, osmolality and pH adjusting agents.

The DNA aptamers or the composition of the invention are suitable for the treatment of skeletal disorders, developmental disorders, and cancers caused by aberrant FGFR1 signaling.

Skeletal disorders are craniosynostoses associated with activating FGFR1 mutations, in particular, Pfeiffer syndrome and osteoglophonic dysplasia (their association with FGFR1 mutation activation is described e.g. in Roscioli et al., 2000, doi: 10.1002/1096-8628(20000703)93: 1; White et al., 2005, doi: 10.1086/427956; Muenke et al., 1994, doi: 10.1038/ng1194-269).

Developmental disorders include, in particular, encephalocraniocutaneous lipomatosis; association of this disorder with activating mutations in FGFR1 was reported in Bennett et al., 2016 (doi: 10.1016/j.ajhg.2016.02.006).

Cancers are tumors of various etiology associated with activating mutations in FGFR1. These tumors include, in particular, breast cancer, colorectal cancer, esophageal carcinoma, gastric cancer, glioblastoma, squamous cell carcinoma, melanoma, astrocytoma, prostate cancer and seminoma which contain mutation in FGFR1, as disclosed in Gallo et al. (2015, https://doi.org/10.1016/j.cytogfr.2015.03.003).

### Brief description of drawings

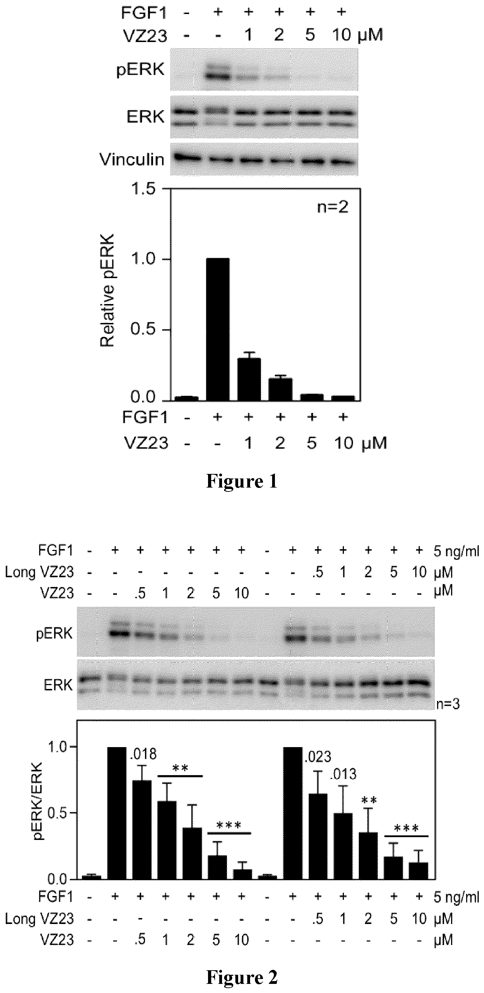

Figure 1: RCS-FGFR1c cells were treated with long VZ23 (SEQ ID NO. 6) for 30 minutes, followed by FGF1 (5 ng/ml) treatment for 1 hour. FGF1-mediated phosphorylation (p) of ERK MAP kinase was determined by western blot. Total ERK and vinculin served as loading controls. Western blot data were quantified and graphed (mean±SD; n, number of independent experiments). Long VZ23 shows an inhibitory effect on FGF1-mediated ERK activation.
Figure 2: Comparison of VZ23 (SEQ ID NO. 2) and long VZ23 (VZ23 with primers, SEQ ID NO. 6). RCS-FGFR1c cells were treated with aptamers for 30 minutes and then treated with FGF1 for one hour. Cells were analyzed for phosphorylated (p) ERK activation by western blot. Total ERK serves as a loading control. pERK signal was quantified and plotted (mean±SD; t-test, **p<0.01, ***p<0.001; n, number of independent experiments).

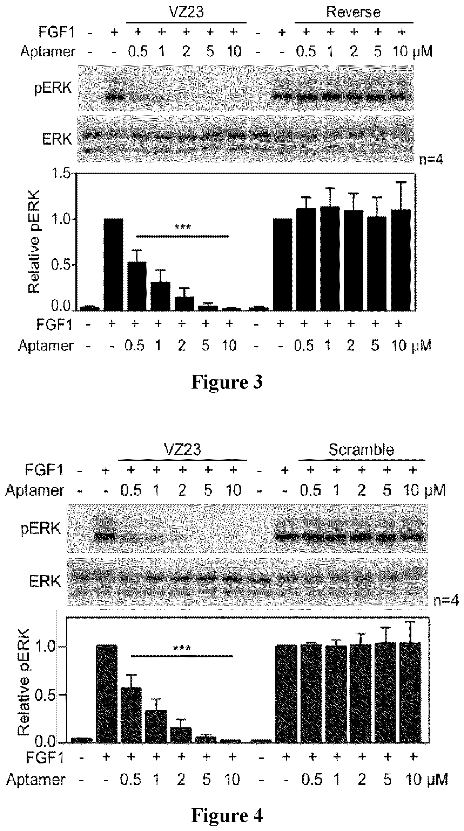

Figure 3: RCS-FGFR1c cells were treated with VZ23 (SEQ ID NO. 2) or reverse aptamer for 30 minutes, followed by FGF1 (5 ng/ml) treatment for 1h. The FGF1-mediated phosphorylation (p) of ERK MAP kinase was determined by western blot. Total ERK served as loading control. Western blot signal was quantified and graphed (mean±SD; ANOVA, *** p<0.001; n, number of independent experiments). VZ23 inhibits FGF1-mediated activation of ERK, reverse aptamer has no effect.
Figure 4: RCS-FGFR1c cells were treated with VZ23 (SEQ ID NO. 2) or scramble aptamer for 30 minutes, followed by heparin (1 µg/ml) and FGF1 (5 ng/ml) treatment for 1h. The FGF1-mediated phosphorylation (p) of ERK MAP kinase was determined by western blot. Total ERK served as loading control. Western blot signal was quantified and graphed (mean±SD; ANOVA, *** p<0.001; n, number of independent experiments). VZ23 inhibits FGF1-mediated activation of ERK, reverse aptamer has no effect.

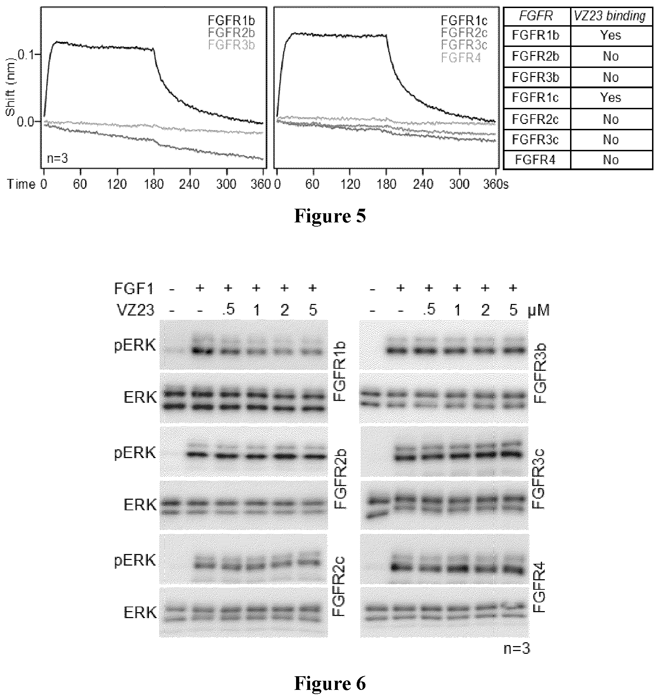

Figure 5: BLI analysis of VZ23 (SEQ ID NO. 2) binding on FGFRs. VZ23 binds to FGFR1b and FGFR1c, but not to the other tested FGFR variants.
Figure 6: VZ23 (SEQ ID NO. 2) inhibits FGF1-mediated activation of ERK in RCS-FGFR1b cells but not in RCS cells expressing FGFR2b, FGFR2c, FGFR3b, FGFR3c and FGFR4.

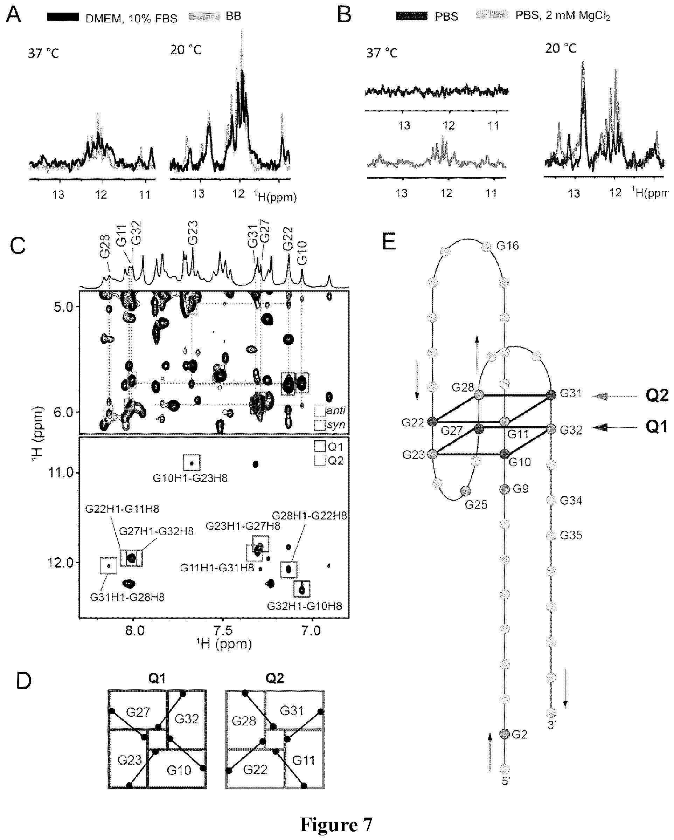

Figure 7: Structural characterization of VZ23 (SEQ ID NO. 2). (A, B) Imino regions of 1D ¹H NMR spectra of VZ23 acquired in DMEM supplemented with 10% FBS, binding buffer (BB), PBS, and PBS supplemented with 2 mM MgCh at 37°C and 20°C. The spectra suggest that VZ23 adopts a non-canonical DNA structure stabilized by Watson-Crick and Hoogsteen base pairs and this structure stability depends on Mg²⁺. (C) Anomeric-aromatic and imino-aromatic regions of ¹H-¹H 2D NOESY NMR spectrum (mixing time 250 ms) of VZ23 were used to assess glycosidic conformations of guanines from G-quartets and intra-quartet H1-H8 NOE connectivities, respectively. (D) G-quartets 1 and 2 (Q1 and Q2) with the corresponding H1-H8 NOE connectivities observed on the ¹H-¹H 2D NOESY spectrum. (E) Schematic presentation of G-quadruplex-based topology adopted by VZ23 with antiparallel orientations of G-strands, long 5'- and 3'-tails, and three edgewise loops, respectively. Residues in G-quartets 1 and 2 (Q1 and Q2) adopting anti- and *syn*-glycosidic conformations are colored light and dark grey, respectively. The antiparallel orientation of the G-strand is emphasized with black arrows.

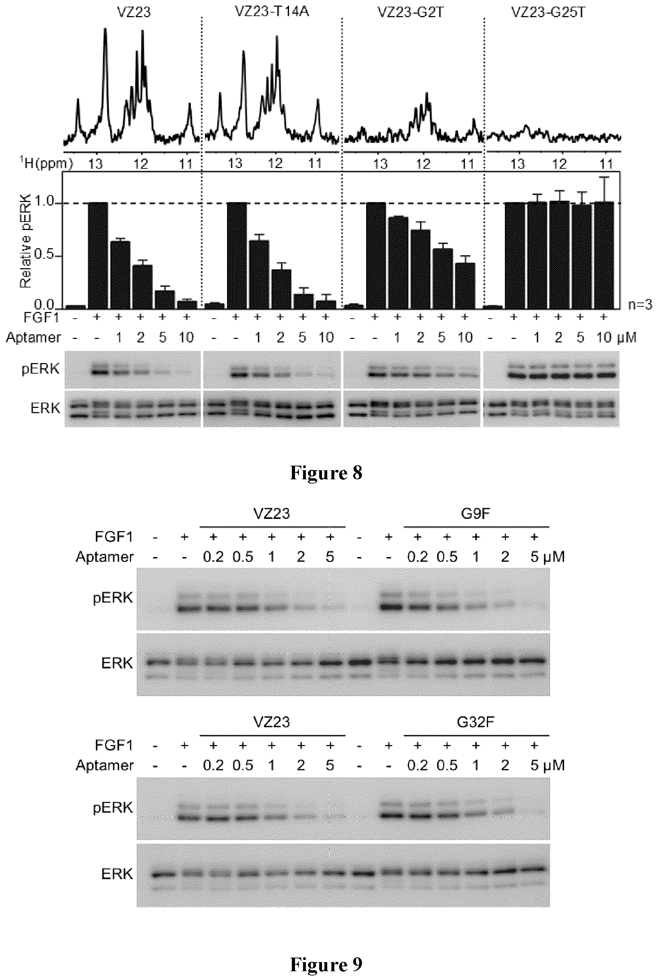

Figure 8: Effect of VZ23 stability on its inhibitory activity. Imino regions of 1D ¹H NMR spectra of VZ23 (SEQ ID NO. 2) mutants T14A (SEQ ID NO. 3), G2T, and G25T. The decreased signal intensities in the mutants' NMR spectra, compared to the spectrum of wildtype VZ23, reflect the impaired capacity of the mutants to fold (upper graph). VZ23-T14A, G2T, and VZ23-G25T inhibitory effect on FGF1-mediated activation of ERK signaling in RCS-FGFR1c cells. pERK signal was quantified and plotted (lower graph, western blot). Collectively, the data show that the stability of the VZ23 structure positively correlates with aptamer inhibitory activity, i.e., the higher the aptamer's stability, the higher its inhibitory effect.
Figure 9: 2'-ara fluorination of VZ23 (SEQ ID NO. 2) at guanosine 9 (G9F; SEQ ID NO. 4) and 32 (G32F; SEQ ID NO. 5) shows comparable inhibitory activity to VZ23. RCS-FGFR1c cells were treated with aptamers for 30 minutes and then treated with FGF1 for one hour. Cells were analyzed for phosphorylated (p) ERK activation by western blot.

### Detailed disclosure of the invention

The aptamers used in the experiments can be prepared using solid-state synthesis based on standard phosphoramidite chemistry or enzymatic synthesis *in vitro* using Taq DNA polymerase.

The following methods were used in studying the properties and biological activity of the DNA aptamers of the invention:
NMR and CD spectroscopy: NMR spectra were measured on Bruker Avance NEO spectrometers (Bruker) at 600, 850 or 950 MHz using triple resonance 5 mm cryogenic probes. Spectra of 50 µM DNA samples in the PBS, binding buffer, or 10% DMEM and 10% D2O were recorded at 20 or 37°C. 1D ¹H NMR spectra were acquired using a 3-9-19 pulse sequence with gradients to suppress the water signal. Spectra were baseline corrected and processed with the exponential apodization function with the line-broadening parameter set to 10 MHz. Imino protons were assigned using ¹⁵N-edited HSQC spectra, while imino-aromatic and anomeric-aromatic NOE connections were identified using ¹H-1H 2D NOESY spectra with 250 ms mixing time. The spectra were acquired and processed with TopSpin v4.3.0 (Bruker, Germany). 1D ¹H and ¹⁵N-edited NMR spectra were analyzed using MestReNova v14.2.2 or v14.3.3 (Mestrelab Research), while the resonances on 2D NOESY spectra were assigned using NMRFAM-Sparky software (UCSF). CD measurements were acquired using Jasco J815 spectropolarimeter (Jasco). Aptamers were refolded before each measurement. CD spectra were obtained in at L220-320 nm. CD melting experiments were carried out in the 4-94°C range at 2°C/min heating rate, with changes in the signal monitored at L 289 nm. The UV absorption spectra for TDS analysis were acquired simultaneously with CD experiments on Jasco J815 spectropolarimeter. Data were processed and plotted using GraphPad Prism 8 software.

Recombinant FGFR production and biolayer interferometry (BLI): Fully glycosylated extracellular domains of human FGFR1b, FGFR1c, FGFR2b, FGFR2c, FGFR3b, FGFR3c and FGFR4 were fused to the Fc domain of IgG1, and purified as described before (Sokolowska-Wedzina et al., 2014, https://doi.org/10.1016/j.pep.2014.03.012). BLI experiments were performed on an Octet K2 (Sartorius AG) using Protein A biosensors. The FGFRs were immobilized on Protein A biosensor, and incubated with VZ23 diluted in PBS. Association and dissociation were monitored for 300s each. The resulting data were analyzed using Data Analysis 11.0 software from ForteBio. Equilibrium dissociation constants (KD) were calculated from fitted saturation binding curves. For competitive BLI experiments, FGFR1c or FGFR1b were immobilized on Protein A biosensors at 5 ug/ml, and measurements were conducted with VZ23 and FGF1 diluted in PBS to 250 nM each. The first association step was measured for VZ23 only and the second association for equimolar mixture of VZ23 and FGF1 followed by dissociation in PBS. Alternatively, second set of measurements was performed with FGF1 only association measured first, repeating the remaining steps. All stages were monitored for 180 s each. The data obtained were analyzed using Data Analysis 11.0 software from ForteBio.

Cell culture and western blot: Rat chondrosarcoma (RCS) cells were propagated in DMEM media, supplemented with 10% FBS and antibiotics (Invitrogen). Recombinant FGF1, FGF2 and FGF4 were from Biotechne, heparin was from Sigma. RCS cells expressing endogenous single FGFR2c, FGFR3c or FGFR4 were generated from wildtype RCS cells where endogenous FGFR1-4 genes were individually targeted by CRISPR-Cas9 to leave only one single FGFR expressed. RCS cells expressing endogenous only FGFR1c (RCS-FGFR1c) were prepared similarly, using the FGFR3/4 double knockout RCS cells. The FGFR1-4 null cells were generated from RCS cells where all four endogenous FGFR genes were targeted by CRISPR-Cas9. The cells expressing single FGFR variant (FGFR1b, FGFR2b or FGFR3b) were generated by stable transfection of FGFR1-4 null cells with vectors containing the individual V5-tagged, full length-human FGFR1-4. A stable integration was achieved by PiggyBac transposase and low FGFR expression was ensured by attenuated CMV promotor, as described earlier (Kimura et al, 2021, https://doi.org/10.1126/scitranslmed.aba4226). The wildtype FGFR1c (Addgene_201106; www.addgene.org/201106/) and FGFR expression vectors were described before (Gudernova et al., 2017, https://doi.org/10.7554/eLife.21536). For western blot, cells were harvested directly into the Laemmli sample buffer; lysates were resolved by SDS-PAGE, transferred onto a PVDF membrane and visualized by chemiluminiscence (ThermoFisher). Western blot signal was quantified in ImageJ (http://imagej.nih.gov/ij/). The following table lists all used antibodies.

| *Protein* | *Source* | *Cat. No.* |
|---|---|---|
| Actin | Cell Signaling | 3700 |
| ERK | Cell Signaling | 9102 |
| pERK^{T202/Y204} | Cell Signaling | 9101 |
| pFRS2^{Y196} | Cell Signaling | 3864 |
| GAB1 | Cell Signaling | 3232 |
| pGAB1^{Y627} | Cell Signaling | 3231 |
| Tubulin | Abcam | ab 11316 |
| Vinculin | Cell Signaling | 13901 |
| mouse IgG | Sigma Aldrich | A6782 |
| rabbit IgG | Sigma Aldrich | A6667 |

The inventors treated RCS-FGFR1c cells with the cognate FGFR1c ligand FGF1, and FGF1-mediated activation of the FGFR1c downstream signaling, RAS-ERK-MAP kinase (ERK pathway), was determined by western blot. Long VZ23 (SEQ ID NO. 6) completely inhibited ERK activity (Fig. 1).

In initial experiments, the 40 nt VZ23 sequence plus two 18 nt flanking primer sequences used for PCR amplification (long VZ23) (sequence ATACCAGCTTATTCAATTGGGATACAGGGCTT TGTCTATGGTGTGGATGGCGGATACCAGATAGTAAGTGCAATCT (SEQ ID NO. 6, primer sequences underlined) showed complete inhibition of FGF 1-mediated activation of the ERK pathway in RCS-FGFR1c cells (Fig. 1). Identical inhibition of FGF1-mediated ERK activation was observed for VZ23 without primer sequences (VZ23; SEQ ID NO. 2) (Fig. 2). No activity was found for reverse or scramble DNA sequences of VZ23 (Figs. 3, 4).

To test the specificity of VZ23 against the FGFR variants, the inventors investigated the interactions of VZ23 with FGFRs using biolayer interferometry (BLI). Recombinant human extracellular domains of FGFR1b, FGFR1c, FGFR2b, FGFR2c, FGFR3b, FGFR3c and FGFR4 fused to the Fc domain of IgG1 were prepared as described (Sokolowska-Wedzina et al., 2014, https://doi.org/10.1016/j.pep.2014.03.012) and immobilized on ProtA sensors. Serial dilutions of VZ23 were analyzed for association with FGFR1a and FGFR1b. Kinetic measurements, followed by global fitting with the 1:1 ligand model and steady-state analysis, showed that VZ23 associates with FGFR1 variants, with K_{D} values of 54.6±5.4 nM for FGFR1b and 162±48.7 nM for FGFR1c. No association of FGFR2b, FGFR2c, FGFR3b, FGFR3c or FGFR4 with VZ23 was detected by BLI (Fig. 5).

Next, the inventors examined the specificity of VZ23 towards FGFR variants in cells. RCS cells expressing only FGFR1b (RCS-FGFR1b), FGFR2b (RCS-FGFR2b), FGFR2c (RCS-FGFR2c), FGFR3b (RCS-FGFR3b), FGFR3c (RCS-FGFR3c) and FGFR4 (RCS-FGFR4) were treated with VZ23 (SEQ ID NO. 2) prior to FGF1 treatment and ERK activation was determined. VZ23 inhibited FGF1-mediated ERK activation in RCS-FGFR1b cells (Fig. 6). Of note, VZ23 did not inhibit FGF1-mediated ERK activation in cells expressing FGFR2b, FGFR2c, FGFR3b, FGFR3c and FGFR4.

The imino region of ¹H 1D NMR spectra of VZ23 (SEQ ID NO. 2) acquired at 20°C and 37°C in binding buffer (BB) and FBS-supplemented DMEM medium showed virtually identical patterns marked by multiple signals between 10-14 ppm (Fig. 7A), indicating that VZ23 adopts non-canonical DNA structure stabilized by Watson-Crick and Hoogsteen base pairs. In contrast, the corresponding NMR spectrum of VZ23 acquired in the PBS buffer at 37°C showed no signals (Fig. 7B), which indicates the structure unfolding under these conditions. Notably, the pattern of the NMR spectrum reminiscent of those observed in the binding buffer and FBS-supplemented DMEM medium was observed in PBS buffer upon adding Mg²⁺ (Fig. 7B), showing that the structure stability depends on this ion. In line with this observation, the CD melting profiles acquired in PBS buffer with and without adding Mg²⁺ revealed that adding 2 mM Mg²⁺ results in about 10°C increase in the structure melting temperature. Altogether, these data highlight a critical role of Mg²⁺ for VZ23 stability at 37°C. The analysis of imino-aromatic connectivities in ¹H-¹H 2D NOESY spectra (Fig. 7C) revealed that in the presence of Mg²⁺, VZ23 adopts an antiparallel G-quadruplex-like folding topology (Fig. 7E).

Remarkably, the stabilization of the VZ23 in the presence of Mg²⁺ ions translates into changes in K_{D} of VZ23-FGFR1 association in the presence of Mg²⁺, which decreased by 45% for FGFR1b (54.6±5.4 vs. 29.8±9 nM with Mg²⁺) and 60% for FGFR1c (162±49.7 vs. 62.4±5.5 nM with Mg²⁺).

To investigate the relationship between the structural stability of VZ23 (SEQ ID NO. 2) and its biological activity, single nucleotide substitutions G2T, T14A (SEQ ID NO. 3) and G25T were introduced into VZ23. Analysis of the NMR spectra of these VZ23 mutants revealed no effect of the T14A substitution on the VZ23 structure, a moderate destabilizing effect of G2T and a significant destabilizing effect of the G25T substitution (Fig. 8A). The relative stability of the VZ23 mutants correlated with their biological activity in RCS-FGFR1c cells. VZ23-G25T showed no inhibitory effect on FGF1-mediated activation of ERK signaling, G2T showed only partial inhibition compared to wildtype VZ23 (Fig. 8B), while VZ23-T14A inhibited FGF1-ERK activity to a similar extent as wildtype VZ23. Altogether, these results emphasize the importance of the secondary structure of VZ23 for its association with FGFR1c and its inhibitory effect in cells.

FGF1 is not the only cognate ligand for FGFR1c, which can be equally activated by FGF2 and FGF4 (Omitz et al., 1996, https://doi.org/10.1074/jbc.271.25.15292). Treatment of RCS-FGFR1c cells with FGF2 or FGF4 resulted in ERK activation comparable to that of FGF1; VZ23-mediated inhibition of FGFR1c signaling was also similar for the three ligands, with IC50 values of 0.57, 0.44, and 0.48 µM for FGF1, FGF2, and FGF4, respectively. These data indicate that VZ23 (SEQ ID NO. 2) does not inhibit FGFR1c signaling in an FGF-specific manner. BLI experiments show that VZ23 does not prevent FGF1 interaction with FGFR1b or FGFR1c. Furthermore, VZ23 can associate with already formed FGF1:FGFR1b and FGF1:FGFR1c complexes, supporting the hypothesis that the VZ23 binding site on FGFR1 is independent of the FGF binding site.

Noteworthy, the chemically modified VZ23 analogs, bearing fluorine at 2'-ara position at guanosine 9 and 32, showed comparable activity to parent VZ23 (Fig. 9).

Overall, the experimental results described herein show that specific and effective inhibition of FGFR1 can be achieved by the DNA oligonucleotide based on SEQ ID NO. 1.

## Claims

1. DNA aptamer having a length of up to 80 nucleotides, preferably up to 40 nucleotides, and being or containing the sequence:
GGGATACAGGGCTXTGTCTATGGTGTGGATGGCGGATACC (SEQ ID NO. 1),
wherein X is T or A, and
wherein one to three deoxynucleotides may optionally be modified by fluorination.

2. DNA aptamer according to claim 1, wherein the deoxyguanosine in position 9 and/or 32 of SEQ ID NO. 1 are replaced by 2'-deoxy-2'-fluoroarabinoside guanosine.

3. DNA aptamer according to claim 1, which is or contains a sequence selected from the group:
GGGATACAGGGCTTTGTCTATGGTGTGGATGGCGGATACC (SEQ ID NO. 2),
GGGATACAGGGCTATGTCTATGGTGTGGATGGCGGATACC (SEQ ID NO. 3),
GGGATACAG_{F}GGCTTTGTCTATGGTGTGGATGGCGGATACC (SEQ ID NO. 4),
GGGATACAGGGCTATGTCTATGGTGTGGATGG_{F}CGGATACC (SEQ ID NO. 5), 
ATACCAGCTTATTCAATTGGGATACAGGGCTTTGTCTATGGTGTGGATGGCGGATACCAG
ATAGTAAGTGCAATCT (SEQ ID NO. 6),
wherein G_{F} represents 2'-deoxy-2'-fluoroarabinoside guanosine.

4. DNA aptamer according to any one of the preceding claims which in the presence of a physiological concentration of Mg²⁺ bind selectively to FGFR1b and FGFR1c but do not bind to FGFR2b, FGFR2c, FGFR3b, FGFR3c or FGFR4, and mediate inhibition of FGFR1b and FGFR1c.

5. A composition comprising at least one DNA aptamer of any one of the preceding claims, at least one pharmaceutically acceptable excipient, and optionally Mg²⁺ ions.

6. DNA aptamers according to any one of claims 1 to 4 or the composition according to claim 5 for use in the treatment of skeletal disorders, developmental disorders, and cancers caused by aberrant FGFR1 signaling.

7. DNA aptamers according to any one of claims 1 to 4 or the composition according to claim 5 for use according to claim 6, wherein the skeletal disorders are craniosynostoses associated with activating FGFR1 mutations, preferably the skeletal disorders are Pfeiffer syndrome or osteoglophonic dysplasia.

8. DNA aptamers according to any one of claims 1 to 4 or the composition according to claim 5 for use according to claim 6, wherein the developmental disorder is encephalocraniocutaneous lipomatosis.

9. DNA aptamers according to any one of claims 1 to 4 or the composition according to claim 5 for use according to claim 6, wherein the cancers are selected from breast cancer, colorectal cancer, esophageal carcinoma, gastric cancer, glioblastoma, squamous cell carcinoma, melanoma, astrocytoma, prostate cancer and seminoma.
